# EUROPEAN PATENT SPECIFICATION

(11) **EP 0 630 648 B1**
(45) Date of publication and mention of the grant of the patent: **07.01.1998**
(21) Application number: 94830299.7
(22) Date of filing: 20.06.1994
(51) Int. Cl.: A61K 31/22

(54) **Use of acyl L-carnitine gamma-hydroxybutyrate for the manufacture of a medicamentfor the treatment of drug addiction**
Verwendung van Acyl-L-Carnitin-gamma-hydroxybutyraten zur Herstellung eines Arzneimittels zur Behandlung der Drogenabhängigkeit
Utilisation de Y-hydroxybutyrates d'acyl-L-carnitines pour la fabrication d'un médicament pour le traitement de la dépendance aux drogues

(30) Priority: 24.06.1993 IT RM930412
(43) Date of publication of application: 28.12.1994
(73) Proprietor: Sigma-Tau Industrie Farmaceutiche Riunite S.p.A., 00144 Roma (IT)
(72) Inventor: Scapagnini, Giovanni, I-90500 Catania (IT); Cavazza, Claudio, I-00186 Rome (IT)
(74) Representative: Fassi, Aldo, Dr.

(56) References cited:
- EP-A- 0 344 704
- EP-A- 0 514 357
- BR. J. EXP. PATHOL. vol. 58, no. 6 , 1977 pages 606 - 615 C. ABU MURAD ET AL. 'Hepatic triglyceride accumulation and the ethanol physical withdrawal syndrome in mice'

## Description

The present invention relates to in a new therapeutic use of acyl L-carnitine esters with gamma-hydroxybutyric acid of formula (I) 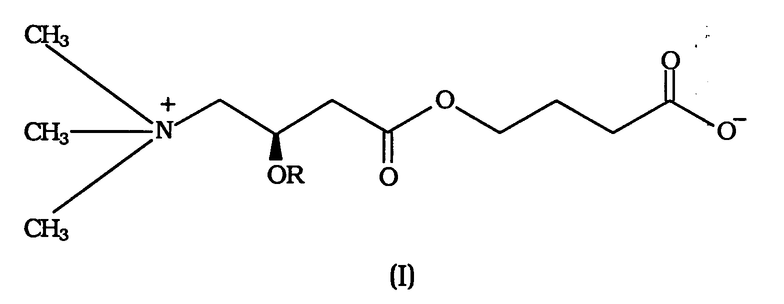 which represents them in the form of inner salts, in which R is linear or branched acyl with from 2 to 5 carbon atoms, preferably acetyl, propionyl, n-butyryl, isobutyryl, valeryl or isovaleryl,
or formula (I') 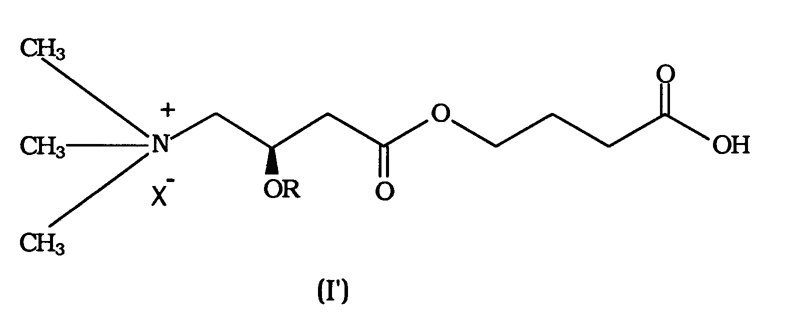 which represents them in the form of salts with pharmacologically acceptable acids, where R has the meaning indicated above and X- is the anion of such an acid.

X- is, for example: chloride; bromide; iodide; aspartate, particularly acid aspartate; citrate, particularly acid citrate; tartrate; phosphate, particularly acid phosphate; fumarate, particularly acid fumarate; glycerophosphate; glucose phosphate; lactate; maleate; particularly acid maleate; orotate; oxalate, particularly acid oxalate; sulphate, particularly acid sulphate; trichloroacetate, trifluoroacetate and methanesulphonate.

The formula (I) or formula (I') esters are used for the preparation of a medicament for the treatment of addiction to psychotropic and narcotic substances such as opiods, amphetamines, cocaine, heroine and morphine. This medicament suppresses withdrawal symptoms in drug addicts (such as tremors, perspiration, hyperreflexia, nausea, anxiety and convulsions), drug addiction and the craving for self-administration of narcotic substances.

The formula (I) and (I') esters are known compounds. In European patent application No. 0 442 850 A1 (published on 21.08.91) their synthesis, physico-chemical characteristics and pharmacological activity in inhibiting neuronal degeneration (as occurring typically in Alzheimer's dementia and in Parkinson's disease) and in the treatment of coma are described.

In European patent application No. 0 514 357 A1 (published on 19.11.92) a further therapeutic use of these formula (I) and (I') esters to produce a drug for the treatment of liver diseases is described. Br. J. Exp. Pathol.; 58(6), 1977, p. 606-615 describes that D,L-Carnitine reduced ethanol withdrawal symptoms. EP-A-344 704 describes that gamma-butyric acid salts are effective in treating alcohol-dependence.

There is no relationship between these known uses and the use envisaged in this invention.

The mechanisms underlying addiction to the various psychotropic and narcotic substances, such as opioids, amphetamines, cocaine, etc., are complex and probably specific to each substance category. Activation of dopaminergic neurons of the mesolimbic system, however, appears to be a process common to the effects of all these substances, playing a major role in bringing about the biochemical and behavioral changes responsible for drug addiction. A great deal of scientific evidence has shown that the chronic abuse of such substances induces an increase in dopamine release and that the onset of the craving, i.e. the compulsive desire and yearning for the drug due to withdrawal of administration of narcotic and pyschotropic agents is, in fact, mainly caused by a reduction in dopamine concentration at neuronal ending level in various areas of the brain.

The substances used to date in the treatment of drug addicts (anaesthetics, benzodiazepaines, etc.) have a non-specific action, often presenting a cross addiction or tolerance with the narcotic substances themselves. The clinical use of these treatments is limited by the occurrence of adverse reactions such as excessive CNS depression and habituation, and by their short duration of action and low safety indices.

All the drugs used to date for the treatment of drug addiction present substantial drawbacks.

Thus, finding a safe, effective drug for the treatment of drug addiction is an objective of major clinical and social importance.

It has now been found that formula (I) and formula (I') esters administered orally or parenterally at non-hypnotic doses suppress withdrawal symptoms and craving in drug addicts or in subjects abusing psychotropic or narcotic substances without causing the above-mentioned adverse or toxic reactions.

Reported here below are details of a number of in-vivo pharmacological studies which demonstrate the activity of one of the compounds as per the invention, namely isovaleryl L-carnitine gamma-hydroxybutyrate.

In the description following here below, VCGB stands for isovaleryl L-carnitine gamma-hydroxybutyrate, GHB for gamma-hydroxybutyric acid, LAC for acetyl L-carnitine and VC for isovaleryl L-carnitine.

### EFFECTS OF VCGB ON HEROIN SELF-ADMINISTRATION BEHAVIOUR IN RATS

This study was conducted in order to establish the effects of VCGB on heroin self-administration behaviour patterns in rats. The action exerted by VCGB was compared with that resulting from the administration of gamma-hydroxbutyrate and acetyl L-carnitine. The experiment was conducted in male Wistar rats weighing approximately 200 g (+ 20 g) housed in groups of 5 per cage for at least two weeks prior to the experimental phase under conditions of constant light-dark alternation at 21°C and fed with standard laboratory feed and water ad libitum.

The heroin self-administration behaviour patterns were studied according to the procedure described by van Ree and de Wied (Ree J.M. van, Wied D. de, 1977, Heroin self-administration is under control of vasopressin. Life Sci., 21:315-320). Briefly, the rats, in which an indwelling jugular cannula had been implanted surgically, were subjected to the experimental session for 3 hours on 5 consecutive days: they were put in soundproofed cells where they were allowed free access to heroin self-administration. The activation of a lever which they were free to use had been programmed to administer an intravenous dose of 0.25 ml of heroin solution (0.125 mg/ml of heroin in saline, pH 7.4) in 15 sec.

The animals were divided into 5 groups, each consisting of five rats. The first group (A) was administered saline solution, the second (B) VCGB, the third (C) GHB, the fourth (D) LAC, and the fifth (E) VC. The administrations of these substances (100 mg/kg) were given daily throughout the test period by the intraperitoneal route. The number of self-administrations of heroin in the space of 3 hours from the third day of the experiment onwards were measured. The results of this behaviour test are given in the table here below:

### Evaluation of the number of heroin self-administrations from the third day of the experiment onwards

| Animal group | 3rd day | 4th day | 5th day |
|---|---|---|---|
| A Saline | 9.1 ± 1.0 | 10.0 ± 1.1 | 9.6 ± 0.9 |
| B VCGB | 6.3 ± 0.5* | 6.8 ± 0.4* | 6.3 ± 0.5* |
| C GHB | 7.5 ± 0.6* | 8.0 ± 0.7* | 9.0 ± 0.6* |
| D LAC | 7.0 ± 0.5* | 9.2 ± 0.5 | 8.9 ± 0.8* |
| E VC | 7.2 ± 0.5* | 9.5 ± 0.5 | 8.8 ± 0.5* |

| | | | |
|---|---|---|---|
| * Significant difference compared to control group (P < 0.05, Dunnet's test for multiple comparisons) | | | |

From the results it can be seen that VCGB has a very long-lasting action in reducing the number of self-administrations, producing an effective reduction in heroin consumption.

### EFFECTS OF VCGB ON ANALGESIA INDUCED BY ACUTE ADMINISTRATION OF MORPHINE

The experiment was conducted in male Wistar rats weighing approximately 250 g (+ 20 g) housed in groups of 5 per cage for at least two weeks prior to the experimental phase under conditions of constant light-dark alternation at 21°C and fed with standard laboratory feed and water ad libitum. The antinociceptive action of morphine was measured by means of the hot-plate test. The test measures the reaction time of an animal placed on a plate heated to a temperature of 51°C. The groups of animals received i.p. injections of morphine (10 mg/kg). The 5 groups were also administered saline solution, VCGB, GHB, LAC or VC, respectively by the intraperitoneal route (100 ml/kg). The evaluation of morphine-induced analgesia in the various groups was done according to the abovementioned test immediately after injection (T 0), and 30 (T 30) and 240 minutes (T 240) after treatment. The effect on morphine-induced analgesia is given in the following table:

| mean latency in sec. | | | |
|---|---|---|---|
| Animal group | T 0 | T 30 | T 240 |
| Saline | 6.0 | 15.0 | 6 |
| VCGB | 6.5 | 14.5 | 6 |
| GHB | 5.5 | 14.5 | 5 |
| LAC | 6.5 | 15.0 | 6 |
| VC | 6.5 | 15.0 | 6 |

From the results obtained it can be seen that none of the substances tested has any significant antagonistic effect on morphine-induced analgesia.

### EFFECTS OF VCGB ON ONSET OF HABITUATION TO THE ANALGESIC ACTION OF MORPHINE

The experiment was conducted in male Wistar rats weighing approximately 250 g (± 20 g) housed in groups of 5 per cage for at least two weeks prior to the experimental phase under conditions of constant light-dark alternation at 21°C and fed with standard laboratory feed and water ad libitum. Morphine addiction was induced in the rats by daily injections for a period of 15 days.

Five different groups of animals also received saline solution, VCGB, GHB, LAC or VC (100 mg/kg i.p.), respectively. The development of habituation to the antinociceptive action of morphine was measured by means of the hot plate test. The test measures the reaction time of an animal placed on a plate heated to a temperature of 51°C.

Evaluation of habituation to the morphine-induced analgesic effect in the various groups was done according to the abovementioned test immediately after injection of an i.p. dose of 10 mg/kg of morphine (T O) and 30 (T 30) and 240 minutes (T 240) after treatment.

| mean latency in sec. | | | |
|---|---|---|---|
| Animal group | T 0 | T 30 | T 240 |
| Saline | 8.0 | 5.0 | 4.0 |
| VCGB | 6.5 | 14.0* | 10.0* |
| GHB | 5.5 | 13.0* | 6.0 |
| LAC | 6.0 | 10.0* | 6.0 |
| VC | 6.0 | 10.0* | 6.0 |

Clearly emerging from the results is the ability of VCGB to reduce habituation to morphine-induced analgesia to an appreciable extent and in a decidedly longer-lasting manner compared to the other substances tested.

### EFFECTS OF VCGB ON WITHDRAWAL SYNDROME DUE TO NALOXONE ADMINISTRATION IN RATS

The experiment was performed in male Wistar rats housed in groups of 5 per cage under conditions of constant light-dark alternation at 21°C and fed with standard laboratory feed and water ad libitum.

After repeated treatment with morphine for a period of 10 days, the rats were all weighed and administered naloxone (1 mg/kg dissolved in saline solution) by subcutaneous injection. The resulting withdrawal syndrome was assessed by measuring the latency of onset of "stereotype jumps" from a circular platform measuring 35 cm in diameter and 70 cm in height. A cut-off time of 900 sec. was used. In 5 groups of animals, naloxone administration was combined with administration of saline, VCGB, GHB, LAC and VC, respectively.

The body weight of the animals was then measured again 1 hour after naloxone injection and the mean loss of body weight was calculated for each group of animals treated.

The results of the experiment are given in the table here below:

| Animal group | Latency of stereotype jumps (sec.) | Weight loss (g) |
|---|---|---|
| Saline | 385 ± 63 | 25.6 ± 1.9 |
| VCGB | 564 ± 69* | 12.3 ± 0.6* |
| GHB | 451 ± 63* | 15.5 ± 0.6* |
| LAC | 465 ± 99* | 18.6 ± 2.1* |
| VC | 480 ± 99* | 16.6 ± 2.1* |

The compounds as per this invention are administered orally or parenterally in any of the usual pharmaceutical forms which are prepared by conventional procedures with which experts in these techniques are fully conversant. These forms include both solid and liquid oral unitary dosage forms such as tablets, capsules, solutions, syrups and the like, and injectable forms such as, for example, sterile solutions for vials and ampoules.

For these pharmacuetical forms the usual solvents, diluents and excipients are used. Optionally, preservative, sweetening and flavouring agents can be added. Non-exclusive examples of such substances are sodium carboxy-methylcellulose, polysorbate, mannitol, sorbitol, starch, avicel, talc and others which are evident to experts in pharmacy.

The dose administered will be decided by the primary care physician, bearing in mind the patient's age, body weight and general condition, on the basis of an appropriate professional assessment.

Though effective results can be noted even at daily doses of from 5 to 8 mg/kg body weight, doses ranging from 10 to 50 mg/kg body weight are optimal. When considered necessary, larger doses can be given, owing to the low toxicity of the compounds covered by the invention. The dosages envisaged, according to the pharmaceutical administration form, are, though not exclusively, the following:
- ampoules :: from 5 to 500 mg
- capsules :: from 15 to 50 mg
- tablets :: from 15 to 500 mg
- oral solutions :: from 15 to 50 mg

## Claims

1. Use of an ester as per formula (I) 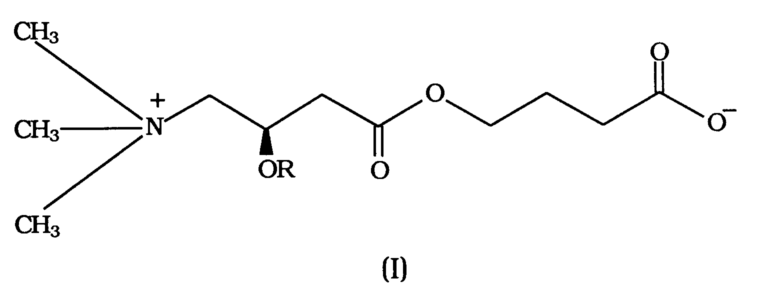 in which R is linear or branched acyl with from 2 to 5 carbon atoms, or as per formula (I') 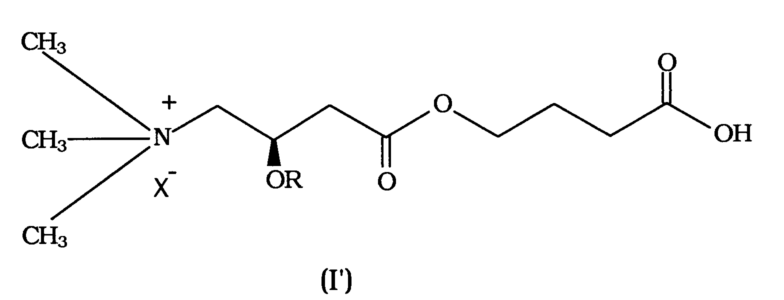 in which R has the meaning indicated above and X⁻ is the anion of a pharmacologically acceptable acid, for the preparation of a drug for the treatment of addiction to psychotropic and narcotic substances.

2. Use according to claim 1 above in which R is chosen from among acetyl, propionyl, n-butyryl, isobutyryl, valeryl and isovaleryl.

3. Use according to claims 1 or 2 above, in which X- is chosen from among chloride; bromide; iodide; aspartate, particularly acid aspartate; citrate, particularly acid citrate: tartrate; phosphate, particularly acid phosphate; fumarate, particularly acid fumarate; glycerophosphate; glucose phosphate; lactate; maleate; particularly acid maleate; orotate; oxalate, particularly acid oxalate; sulphate, parti-cularly acid sulphate; trichloroacetate, trifluoroacetate and methanesulphonate.

## Patentansprüche

1. Verwendung eines Esters mit der Formel (I) 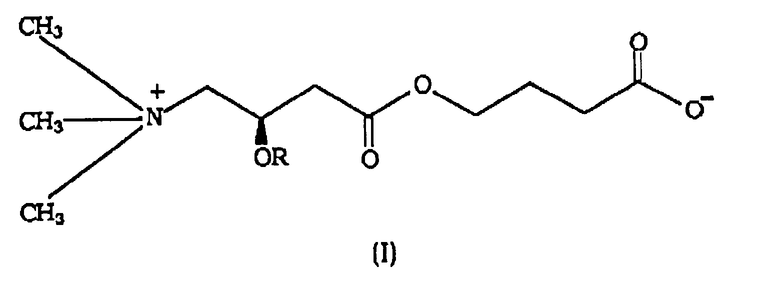 worin R ein lineares oder verzweigtes Acyl mit 2 bis 5 Kohlenstoffatomen ist, oder mit der Formel (I') 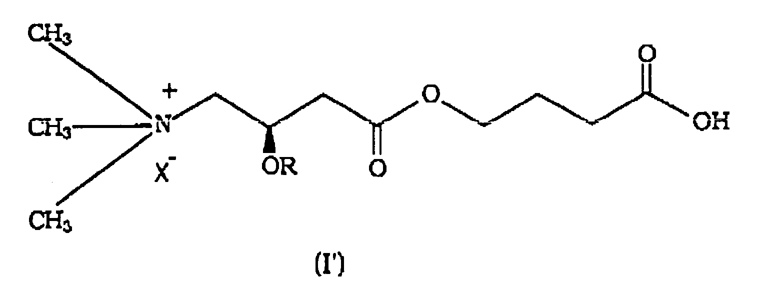 worin R die gleiche Bedeutung wie oben angegeben aufweist und X⁻ das Anion einer pharmakologisch akzeptablen Säure ist, zur Herstellung eines Medikamentes zur Behandlung der Sucht nach psychotropen und narkotischen Substanzen.

2. Verwendung nach Anspruch 1, worin R ausgewählt ist aus Acetyl, Propionyl, n-Butyryl, Isobutyryl, Valeryl und Isovaleryl.

3. Verwendung nach den Ansprüchen 1 oder 2, worin X⁻ausgewählt ist aus Chlorid; Bromid; Iodid; Aspartat, insbesondere saures Aspartat; Citrat, insbesondere saures Citrat; Tartrat; Phosphat, insbesondere saures Phosphat; Fumarat, insbesondere saures Fumarat; Glycerophosphat; Glucosephosphat; Lactat; Maleat, insbesondere saures Maleat; Orotat; Oxalat, insbesondere saures Oxalat; Sulfat, insbesondere saures Sulfat; Trichloroacetat; Trifluoroacetat und Methansulfonat.

## Revendications

1. Utilisation d'un ester de formule (I) 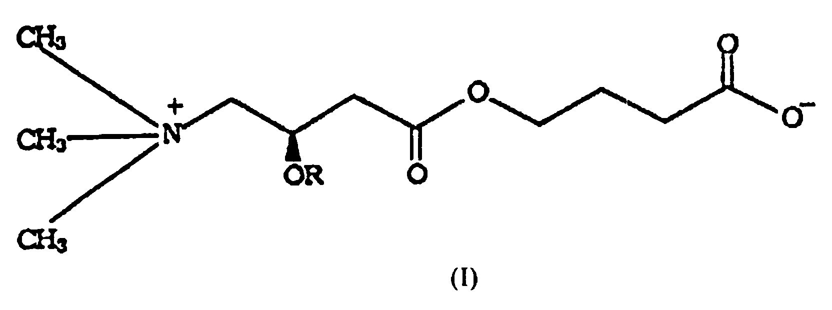 dans laquelle R est un groupement acyle linéaire ou branché de 2 à 5 atomes de carbone, ou de formule (I') 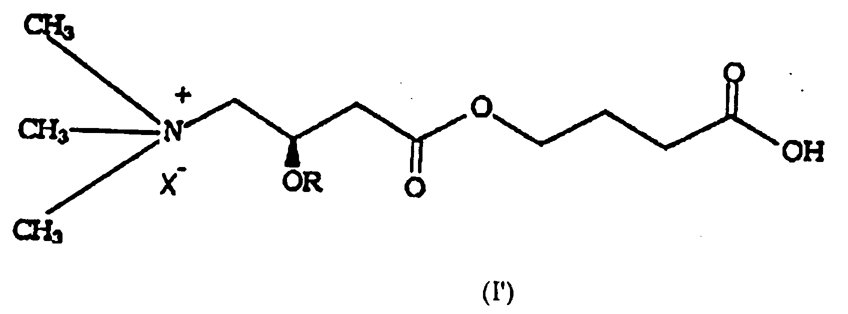 dans laquelle R a la définition indiquée ci-dessus et X⁻ est l'anion d'un acide pharmacologiquement acceptable, pour la préparation d'un médicament destiné au traitement des dépendances à des substances narcotiques et psychotropiques.

2. Utilisation selon la revendication 1 ci-dessus dans laquelle R est choisi parmi les groupements acétyle, propionyle, n-butyryle, isobutyryle, valéryle et isovaléryle.

3. Utilisation selon l'une des revendications 1 ou 2 précédentes dans laquelle X⁻ est choisi parmi les chlorure ; bromure ; iodure ; aspartate, en particulier aspartate acide ; citrate, en particulier citrate acide ; tartrate phosphate, en particulier phosphate acide ; fumarate, en particulier fumarate acide ; glycérophosphate ; phosphate de glucose ; lactacte ; maléate ; en particulier maléate acide ; orotate ; oxalate, en particulier oxalate acide ; sulfate, en particulier sulfate acide ; trichloroacétate, trifluoroacétate et méthanesulfonate.
